(19) Europäisches Patentamt
European Patent Office
Office européen des brevets

(11) **EP 1 754 495 A2**

(12) **EUROPEAN PATENT APPLICATION**

(43) Date of publication:
**21.02.2007 Bulletin 2007/08**

(51) Int Cl.:
*A61L 31/08* (2006.01)    *A61L 31/14* (2006.01)

(21) Application number: **06015856.5**

(22) Date of filing: **29.07.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **19.08.2005 JP 2005238779**

(71) Applicant: **SUN MEDICAL TECHNOLOGY RESEARCH CORPORATION**
**Suwa-shi**
**Nagano (JP)**

(72) Inventors:
• **Yamazaki, Kenji**
**Tokyo (JP)**

• **Kitazume, Toshikatsu**
**No 106 Residence Misuzu**
**Nagano (JP)**
• **Hirata, Tomoko**
**No 205 Residence Misuzu**
**Nagano (JP)**
• **Fujii, Tetsuya**
**Nagano (JP)**
• **Aizawa, Nobuaki**
**Nagano (JP)**

(74) Representative: **Nunnenkamp, Jörg et al**
**Andrejewski, Honke & Sozien**
**Patentanwälte**
**Theaterplatz 3**
**45127 Essen (DE)**

(54) **Sheet-like covering member used for implant medical device**

(57)    The aforementioned object can be achieved with the sheet-like medical covering member of the present invention.

Using a sheet-like polymeric material having a characteristic physical structure that has a large specific surface area, as well as high flexibility, the material further being modified by a hydrophilic surface treatment, and/or by a coating treatment with hydroxyapatite or chitosan, the sheet-like medical covering member of the present invention has high biocompatibility, bio-safety, flexibility, and superior adhesiveness to cells or other tissues, while such a member can be sterilized. The surface of a medical device attached inside a living body and/or to a percutaneous site is covered with the above described covering member, so as to prevent bacteria or outside foreign matter from invading the body, and/or so as to prevent expansion of the infection developed around the interface between the medical device and body tissues in a living body.

FIG.6

EP 1 754 495 A2

## Description

Field of the Invention

The present invention relates to a sheet-like medical covering member, which covers the surface of a medical device attached inside a living body and/or to a percutaneous site, and thereby allows the medical device to be fixed inside a living body and/or to a percutaneous site, so as to allow the medical device to stably stay there for a long period of time, wherein the sheet-like medical covering member is modified by a hydrophilic surface treatment, and/or by a coating treatment with calcium phosphate ceramics or with a composition comprising a biopolymer.

Description of the Related Art

Conventionally, in a treatment using a medical device attached inside a living body or to a percutaneous site, and in particular, a medical device that is fixed inside a living body or to a percutaneous site over a long period of time, such a medical device has been largely problematic in that infectious diseases develop in body tissues, skin tissues, or at a percutaneous site, with which the above medical device is allowed to come into contact. This is because, since the adhesive strength between the surface of a medical device and the tissues near the epidermis is weak at a percutaneous site, separation easily occurs between the medical device and the tissues, and bacteria enter the skin tissues through such an exfoliated portion, and can cause an infectious diseases. Once an infectious disease is developed at a percutaneous site, such infection is spread to other body tissues, resulting in a high risk of developing serious complications such as septicemia. Even in the case where invasion of bacteria into a percutaneous site is prevented, or in the case of using an implantable medical device that is embedded into a body and does not have a percutaneous portion, there may be times when a small amount of bacteria remain in the body. This could occur for several reasons, such as unintended contamination occurring during an operation. At the interface between a medical device and body tissues, adhesion slowly occurs, or adhesive strength is insufficient, due to a foreign body reaction or the like. Due to insufficient immunological functions, such an interface can easily become a nest for the growth of bacteria caused by remaining bacteria. Thus, such an interface has been problematic in that it easily develops infectious diseases.

Accordingly, there is an urgent need to develop a medical covering member, which covers the surface of a medical device attached inside a living body and/or to a percutaneous site, and particularly a medical device that is fixed inside a living body and/or to a percutaneous site for a long period of time, and which rapidly brings on strong adhesion of the above medical device to tissues inside the living body and/or at the percutaneous site, so as to completely isolate the inside of the living body from outside elements, or so as to prevent the expansion of infection around the interface between the medical device and body tissues in a living body. The above described medical covering member differs from materials attached on the skin for a certain short period of time for the purpose of healing a wound, such as an skin-sticking sheet for medical use. The above covering member is intended to be used for covering a medical device that is fixed inside a living body or to a percutaneous site over a long period of time. Thus, it is necessary for the covering member to be stably maintained over a long period of time, as with the above described medical device. Accordingly, in order to completely exert its functions, the above described medical covering member should satisfy at a high level all the following conditions: (i) biocompatibility, (ii) bio-safety, (iii) adhesiveness to cells or other tissues, (iv) flexibility for covering a medical device depending on the configuration thereof, and (v) possibility of being sterilized for the use thereof in a living body.

To date, many materials have been attempted to be used as base materials for medical purposes. Among them, as materials satisfying the aforementioned flexibility or the like, a nonwoven fabric, a woven fabric, and a knitted fabric, each of which is a fiber assembly, have been used. Japanese Patent Laid-Open No. 2004-97267 describes a cuff material having a porous three-dimensional network structure, which exists in the above fiber assemblies to improve the adhesiveness between the material and body tissues.

However, these materials have flexibility, but do not exert sufficient biocompatibility, bio-safety, and adhesiveness to body tissues. The cuff material described in Japanese Patent Laid-Open No. 2004-97267 has a certain degree of improved adhesiveness to body tissues, because of the effects of the physical structure thereof. However, it is difficult to say that its adhesiveness to body tissues is sufficient.

In order to obtain biocompatibility and bio-safety, the surface of an organic polymer used as said material has conventionally been subjected to a certain surface treatment, or the surface of such a material has been subjected to a certain coating treatment, in the medical field.

A treatment for imparting hydrophilicity to the surface of a material has generally been known as a surface treatment on an organic polymeric material. As a coating treatment on the surface of such a material, coating with hydroxyapatite, chitosan, or the like has been known.

Since hydroxyapatite has excellent bioactivity, attempts have been made to densely and uniformly form such hydroxyapatite on the surfaces of various types of medical materials, such that the hydroxyapatite is allowed to strongly bind to the material. For example, Japanese Patent Laid-Open No. 6-293504 and Japanese Patent Laid-Open No. 2004-283324 describe a method for coating the surface of an organic polymer such as a polyester with hydroxyapatite. In addition, Japanese Patent Laid-Open No. 6-293505 describes a method for establishing a bioactive hydroxyapatite film on the surface of a polymeric material, on which an oxygen plasma treatment has previously been performed, by immersing the above material in an aqueous solution containing calcium ions and phosphate ions. Japanese Patent Laid-Open No. 6-293507 describes a method for establishing a hydroxyapatite film on the surface of a polymeric material, on which an alkali treatment has previously been performed, by immersing the above material in an aqueous solution containing calcium ions and phosphate ions. Moreover, as a method for producing a hydroxyapatite complex, Japanese Patent Laid-Open No. 2000-327314 describes a method for producing a hydroxyapatite complex, which comprises a step of alternately immersing a material, at least the surface of which has become hydrophilic, in a calcium solution that contains calcium ions but does not substantially contain phosphate ions, and in a phosphoric acid solution that contains phosphate ions but does not substantially contain calcium ions, so as to generate and immobilize hydroxyapatite at least on the surface of the above material.

Chitosan is a deacetylated product of chitin obtained from Crustacea. It has been known that a large amount of chitosan exists in the nature, and that it is biodegradable and exhibits antiallergic and antibacterial properties. Japanese Patent No. 2579610 describes an *in vivo* filler that consists of a complex of a nonwoven fabric formed by mixing a polyester and other components at a certain ratio and chitosan. Japanese Patent Laid-Open No. 7-258972 and National Publication of International Patent Application No. 9-511666 describe a complex material consisting of chitosan and a protein material such as silk fibers, and a medical device consisting of chitosan and fabric, respectively. Furthermore, Japanese Patent Laid-Open No. 2004-131600 describes: a method for coating the surface of a synthetic polymer molded product with a chitosan compound, which comprises subjecting the surface of the molded product to a plasma treatment and then applying a chitosan compound solution thereto; and a chitosan-coated molded product obtained by the above method.

A medical material obtained by the hydrophilic surface treatment of a common polymeric material and the medical materials described in Japanese Patent Laid-Open No. 6-293504, No. 2004-283324, No. 6-293505, No. 6-293507, No. 2000-327314, and No. 2004-131600, are improved to a certain extent in terms of biocompatibility, bio-safety, and antibacterial properties, by the effects of a surface treatment or coating treatment performed on the surface thereof. However, when a common polymeric material, and particularly, a film- or plate-like polymeric material is used, it is problematic in that flexibility depending on the configuration of a medical device cannot be obtained, and in that cell adherent properties (adhesive properties) sufficient for preventing infectious diseases or the like cannot be obtained in terms of adhesiveness to body tissues.

Moreover, the complex of chitosan and a polyester or the like described in Japanese Patent No. 2579610 has been improved in terms of flexibility, adhesiveness to cells or other tissues, etc. However, since this complex is produced by mixing chitosan with other fibers, it does not mean that 100% of the surface of the obtained fibers is coated with chitosan. Thus, the frequency of allowing chitosan to come into contact with body tissues is not necessarily high, and thus, there is still room for improvement in terms of biocompatibility and bio-safety. Since the complex of a protein material and chitosan described in Japanese Patent Laid-Open No. 7-258972 comprises a protein as a main component, it cannot obtain high stability and physical strength in a living body. This complex is also problematic in that the material is deteriorated by a sterilization operation or the like conducted before use. In the case of the knitted fabric coated with chitosan, described in the National Publication of International Patent Application No. 9-511666 also, since its production process does not include a coating treatment by immersion and the subsequent neutralization and immobilization operations, it becomes difficult to obtain a thin uniform coating layer. Thus, this fabric is problematic in terms of low stability and physical strength in a living body.

As mentioned above, conventional medical materials that have widely been used exhibit their effects to a limited extent, depending on their functions. However, such conventional medical materials have not yet satisfied all the following conditions simultaneously: (i) biocompatibility, (ii) bio-safety, (iii) adhesiveness to cells or other tissues, (iv) flexibility for covering a medical device depending on the configuration thereof, and (v) possibility of being sterilized for the use thereof in a living body. In particular, in order to achieve high flexibility and adhesiveness to cells or other tissues, a base material used for the sheet-like medical covering member of the present invention comprises a fibrous material, a porous material or the like, which has a large specific surface area. With regard to a hydrophilic treatment and/or a coating treatment performed on the base material having the aforementioned properties, it has been difficult to uniformly perform the above treatments on all the portions of the base material, and thus, the effects obtained by the conventional hydrophilic and/or coating treatments have been insufficient. On the other hand, when the aforementioned treatments were performed on the above base material used for the covering member such that the base material exerted sufficient effects, problems such as a decrease in physical strength, deterioration, or a decrease in flexibility of the base material occurred. Thus, it has been difficult to sufficiently perform necessary treatments where the original properties of the base material were not impaired. Accordingly, when a medical device is covered, for example, when a tubular material such as a driveline is covered, if a material that has been subjected to a surface treatment or a coating treatment by conventional techniques is used, such a covering member is problematic in that it loses flexibility and gets hardened, in that the coating is easily exfoliated, or in that complete adhesiveness to cells or other tissues cannot be obtained. Hence, it has been extremely difficult to provide a covering member, which satisfies all the aforementioned conditions, and covers a medical device that is fixed inside a living body or a percutaneous site over a long period of time.

It is an object of the present invention to provide a sheet-like medical covering member, which is obtained by subjecting the surface of a sheet-like polymeric material having a characteristic physical structure having a large specific surface area, as well as high flexibility to a hydrophilic treatment, and/or a coating treatment with calcium phosphate ceramics or a composition comprising a biopolymer, without impairing the original properties of the above material; wherein the surface of a medical device attached inside a living body and/or to a percutaneous site is covered with the above described sheet-like medical covering member, so as to prevent bacteria or outside foreign matter from invading the body, and/or so as to prevent expansion of the infection developed around the interface between the medical device and body tissues in a living body.

FIG. 1 includes SEM (scanning electron microscope) micrographs showing the sheet-like medical covering members obtained in Examples 1 and 2 and Comparative Example 1;
FIG. 2 includes SEM micrographs showing the sheet-like medical covering members obtained in Examples 3, 5, and 7;
FIG. 3 includes SEM-EDX (scanning electron microscope combined with the energy dispersive X-ray microanalysis) micrographs showing the sheet-like medical covering members obtained in Examples 3, 5, and 7;
FIG. 4 shows the results of the X-ray diffractometry of the sheet-like medical covering member obtained in Example 3;
FIG. 5 includes SEM micrographs showing the sheet-like medical covering members obtained in Examples 4, 6, and 8; and
FIG. 6 shows the results of a cell adhesion and cell growth test performed on the sheet-like medical covering members obtained in Examples 1 to 8 and Comparative Example 1.

The sheet-like medical covering member of the present invention can be used for covering the surface of a medical device, which is attached to a body by being perforated through the skin. At that time, the medical device may directly be covered with the present sheet-like medical covering member, or it may be covered with the present sheet-like medical covering member by adhering the covering member to the surface of the medical device using an adhesive or the like. Moreover, depending on the material or configuration of the medical device, after the medical device has been covered with the present covering member, directly or using an adhesive or the like, the covering member is sewn on the surface of the medical device using a thread such as a thread made from polypropylene, so that the covering member can completely be fixed on the medical device. Examples of a medical device for which the sheet-like medical covering member of the present invention is used may include a percutaneous driveline for an artificial heart, or a percutaneous catheter tube used for peritoneal dialysis, an artificial respirator, central venous hyperalimentation, enteral feeding therapy, etc.

A material comprising an organic polymer as a main component can be used as a material for a base material used for the covering member of the present invention. Such an organic polymer includes both natural polymers and synthetic polymers. Examples of a natural polymer may include collagen or hyaluronic acid. Preferred examples may include silk and cotton cellulose, which do not exhibit acute biodegradation. Examples of a synthetic polymer may include polyester, polyolefin, fluorocarbon resin, polyethylene, polypropylene, polystyrene, polyamide, polyimide, polysiloxane, polyether, or polycarbonate. Moreover, examples of a polyester polymer may include polyethylene terephthalate, polybutylene terephthalate, or polyethylene-2,6-naphthalate.

Additives such as a stabilizer used against heat, light, oxidation or the like, a crosslinker, a vulcanizing agent, a dye, a filler, a reinforcer, a plasticizer, or a pigment, may be added to the above polymeric material, to such an extent that such additives do not impair bio-safety.

As a base material of the covering member comprising the aforementioned organic polymer as a main component, a flexible sheet-like material is preferably used. Taking into consideration the compatibility, and adhesiveness to body tissues, namely, to cells, by means of increasing the surface area of the sheet-like covering member that is allowed to come into contact with the cells, the sheet-like base material is preferably a fibrous material, a porous material, or a material having a surface with many asperities. Further, the specific surface area of the sheet-like base material is preferably 100 $cm^2/g$ or more, more preferably 300 $cm^2/g$ or more, and most preferably 1000 $cm^2/g$ or more.

For example, the sheet-like base material to be used in the present invention has a specific surface area of 100 $cm^2/g$ to 10000 $cm^2/g$. In this case, the specific surface area is measured by a method wherein it is calculated using a mathematical principle, based on its typical geometrical configuration.

According to said method, for example, if the sheet-like base material is a fibrous material, the geometrical configuration of an element thereof can be assumed to be cylindrical. The typical diameter of the cylinder can be measured by an optical method or an SEM (Scanning Electron Microscopy) imaging method, or the like. For example, the specific surface area of a nonwoven fabric, which is made of polyester fibers having the diameter measured by the SEM imaging method of 15 micrometers and the density of 1.3 g/cm$^3$, can be thus calculated by the following formula,

$$\text{SSA (cm}^2\text{/g)} = (\pi \times D \times L) / (\pi \times (D/2)^2 \times L \times \rho) = 4/(D \times \rho)$$

[where SSA is a specific surface area (cm$^2$/g); $\pi$ is the ratio of the circumference to diameter; D is the diameter (cm); L is the length of the cylinder (any provisional value may be used due to it being cancelled in the calculation); and $\rho$ is the density of the base material (g/cm$^3$)]

and the resulting specific surface area is about 2000 cm$^2$/g.

Moreover, for example, if the sheet-like base material has an odd-shaped configuration or a random-shaped configuration, a simulated configuration to an element of the base material is able to be designed by means of visual simulative drawing using a three-dimensional CAD (Computer Aided Design) system, or the like. The volume and the surface area of the sheet-like base material can be calculated by integration based on the resulting three-dimensional data of the said simulated configuration. In this case also, the specific surface area of the base material can be analogously calculated by taking its density into account.

Furthermore, the sheet-like base material having a specific surface area of more than 10000 cm$^2$/g can be used in the present invention. In this case, the specific surface area can be conveniently measured by a usual BET method.

Examples of a fibrous material may include a woven fabric, a nonwoven fabric, or a knitted fabric. Examples of a porous material may include Polymeric Foams and e-PTFE. Examples of a material having a surface with many asperities may include an embossed sheet or a micro-embossed sheet. In addition, taking into consideration the characteristics of a sheet-like covering member that covers the surface of a medical device depending on the configuration thereof, in order to not lose flexibility, the thickness of the sheet-like base material is preferably 5 mm or shorter, and more preferably 2 mm or shorter. For example, the sheet-like base material to be used in the present invention may have a thickness of 0.1 mm to 2 mm. The conformation of the sheet-like base material may be a textile fabric, a knitted fabric, or a nonwoven fabric, which is produced from fibers made from the aforementioned polymer, such as polyester fibers, polyolefin fibers, or fluorocarbon resin fibers.

In the method for producing the sheet-like medical covering member of the present invention, a treatment of imparting hydrophilicity to the surface of a base material used for the covering member to such an extent that the surface has substantially sufficient hydrophilicity, or a treatment of coating the above surface with calcium phosphate ceramics or a composition comprising a biopolymer, is performed.

Examples of such a treatment of imparting hydrophilicity to the above base material in the present invention may include a plasma treatment, an alkali reduction treatment, ultraviolet ray irradiation in the presence of ozone, or a dry oxidation treatment such as corona discharge treatment.

Various types of plasma treatment devices can be used in a plasma treatment. Since the base material of the sheet-like medical covering member of the present invention has a filamentous or porous structure to provide flexibility, a device using a method for applying an activated gas uniformly dispersing as active species by gas diffusion mechanism, which is even effective for uniform treatment of the articles, is preferable, rather than a processing mechanism involving irradiation of radioactive ray or ions having a directional non-uniformity. When oxygen plasma is used for example, it is particularly preferable to use a plasma treatment device in which the ground electrode side is established at the bottom of a chamber such that the ground electrode is relatively electropositive (anodic) to the upper electrode (cathode), and such that the material to be treated disposed near the lower electrode is mainly treated by electrically neutral active species such as free radicals. Thus, using such a plasma treatment device, namely, a plasma treatment device configured such that the main portion of active plasma species substantially having no electric charge plays a dominant role in a denaturing process mechanism for treating the material, it becomes possible to sufficiently perform the necessary treatment on a base material used for the covering member of the present invention having the said structural characteristics, without impairing the original properties of the above base material, so that the inside of a void in the base material becomes uniform. Examples of gas used for such a plasma treatment may include argon, oxygen, or ammonia gas. Particularly preferably, the treatment is carried out in an oxygen-containing atmosphere. The time required for the treatment is generally between 1 and 30 minutes. However, in order to promote a sufficient surface treatment reaction and, at the same time, to prevent a loss of the original properties of a base material due to an excessive reaction, the time required for the treatment is preferably between 2 and 20 minutes, and more preferably 2.5 and 10 minutes. The surface of a polymeric base material, and particularly, a base material having two faces, such as a sheet-like polymeric base material, is generally treated on each of the front and back sides thereof. In the aforementioned particularly preferred plasma treatment device also, in order to avoid the non-uniformity of a surface treatment state at close range with an electrode, each of the front and back sides is preferably exposed to oxygen plasma, and particularly to oxygen radicals. In order to enable electric discharge and perform a uniform plasma treatment in the above plasma treatment, the partial pressure of gas is generally between 5 and 500 Pa. In order to enable stable electric discharge and effectively provide the effects of the treatment within a certain period of time, it is preferably between 10 and 200 Pa, and more preferably between 20 and 100 Pa.

An alkali reduction treatment is carried out by treating a base material used for the covering member with an alkaline solution. Examples of an alkaline solution used for the alkali reduction treatment may include an aqueous sodium hydroxide solution or an aqueous potassium hydroxide solution. Of these, an aqueous sodium hydroxide solution is preferable. The concentration of the solution is preferably between 0.5 and 5.0 N, more preferably between 0.8 and 3.0 N, and most preferably between 1.0 and 2.0 N. Other conditions may be adjusted as necessary. The reaction temperature is between 30°C and 95°C, preferably between 70°C and 90°C, and more preferably between 75°C and 85°C. The reaction time is between 10 and 120 minutes, and preferably between 30 and 60 minutes.

An ultraviolet ray irradiation treatment and a dry oxidation treatment can be carried out by methods known to those skilled in the art. Conditions applied for such treatments are not particularly limited, as long as the surface of a base material becomes hydrophilic under such conditions.

Furthermore, an example of a treatment for coating the surface of a base material used for the covering member of the present invention with calcium phosphate ceramics may be a coating treatment with calcium phosphate ceramics mainly comprising hydroxyapatite having bio-safety. Examples of such a coating treatment method using calcium phosphate ceramics may include a solution immersion method, a suspension immersion method, or a solution alternate immersion method. In terms of the rapid rate and high efficiency of formation of hydroxyapatite, the suspension immersion method and the solution alternate immersion method are preferable. In order to obtain a thin uniform coating layer, the solution alternate immersion method is particularly preferable.

In the solution immersion method, a base material used for the covering member is immersed in a solution containing calcium ions and phosphate ions.

As described in Japanese Patent Laid-Open No. 2000-327314, in the solution alternate immersion method, a base material used for the covering member is alternately immersed in a calcium solution and a phosphoric acid solution. A series of operations including immersion of the base material in both solutions and washing between the immersions is defined as 1 cycle, and such a cycle is repeated for a suitable number of cycles. Examples of a calcium solution used for the coating treatment may include an aqueous and a Tris buffer solution containing calcium chloride, calcium acetate, or the like. The concentration of calcium ions contained in such a solution is preferably between 10 and 500 mmol/l, and more preferably between 100 and 300 mmol/l. The pH of such a calcium solution is not limited. It is preferably between pH 6 and 10, and more preferably between pH 7 and 8.

Examples of a phosphoric acid solution used for the coating treatment may include an aqueous and a Tris buffer solution containing dibasic sodium phosphate, disodium hydrogen phosphate, or the like. The concentration of phosphate ions contained in such a solution is preferably between 10 and 500 mmol/l, and more preferably between 100 and 150 mmol/l. The pH of such a phosphoric acid solution is not limited. It is preferably between pH 6 and 10, and more preferably between pH 7 and 8. A washing solution used for washing the aforementioned base material is not particularly limited. Distilled water is preferable.

The temperature applied when the above base material is immersed in the aforementioned calcium solution, phosphoric acid solution, and washing solution, is preferably between 25°C and 40°C, and more preferably between 36°C and 39°C. Each of the times required for immersing the above base material in a calcium solution and a phosphoric acid solution, and for immersing the above base material in a washing solution is between 10 and 600 seconds, preferably between 30 and 480 seconds, and more preferably between 60 and 300 seconds, per cycle. The number of cycles for repeating the aforementioned series of operations is between 1 and 10 times, and preferably between 2 and 6 times. When the base material is immersed in each of the solutions, a solution attached on said material is sufficiently removed, and thereafter, it is immersed in the next solution. As the number of cycles increases, the above solutions and the washing solution become cloudy. Thus, if 5 or more cycles are repeated, such solutions and the washing solution are exchanged with fresh solutions, as appropriate.

In the suspension immersion method, a base material used for the covering member is immersed in a suspension comprising powders of a hydroxyapatite-calcined body. The type of a powdery hydroxyapatite-calcined body used herein is not particularly limited. In order to obtain a uniform coating layer comprised of a large number of fine particles, a hydroxyapatite-calcined body has a mean particle size of preferably 3 micrometers or less, and more preferably 1 micrometer or less.

In all of the coating treatment methods using calcium phosphate ceramics, a covering member, on which a coating treatment has been performed, is dried in a dryer, the temperature of which is adjusted between 40°C and 70°C, and preferably between 50°C and 60°C. The covering member is then immersed in distilled water, followed by ultrasonic washing (approximately, 1 minute × 3 times). Thereafter, the covering member is dried again in a dryer, the temperature of which is adjusted between 40°C and 70°C, and preferably between 50°C and 60°C.

A mean thickness calculated using the weight of a coating layer comprising calcium phosphate ceramics formed by the aforementioned method can be selected as appropriate, depending on the type of base material used, the shape thereof, the form thereof, or the usage thereof. In order to sufficiently exert the coating effects when the covering member covers a medical device, and also to not lose the original properties of the base material such as flexibility, such mean thickness is preferably between 0.001 and 0.5 micrometers, more preferably between 0.002 and 0.2 micrometers, and most preferably between 0.003 and 0.1 micrometers. The coating layer comprising calcium phosphate ceramics formed by the aforementioned method preferably has a main X-ray diffraction strength at 26 degrees and 32 degrees of the $2\theta$ value in X-ray diffractometry. The term "mean thickness of the coating layer formed by the coating treatment using hydroxyapatite-containing calcium phosphate ceramics" is used herein to mean the mean thickness of the coating layer that is calculated using the weight and a specific surface area of the layer attached. Thus, the term expresses an equalized thickness of the coating layer comprising unevenly attached particles with many asperities observed under an actual SEM. The weight of the layer attached that is a base for calculating such a mean thickness is obtained by strictly weighing a sample. However, when it is difficult to calculate a mean thickness from such a weight because the coating layer is extremely thin, such a weight of calcium phosphate ceramics attached can be calculated using the calcium- or phosphorus-specific X-ray intensity measured with a fluorescent X-ray spectrometer.

As a treatment for coating a base material used for the covering member of the present invention with a composition containing a biopolymer, it is preferable to coat the base material with a composition containing a biopolymer that is positively chargeable in water, and it is more preferable to coat the base material with a composition containing chitin or chitosan that is a chitin derivative. Examples of such a biopolymer that is positively chargeable in water may include: natural polymers such as chitin or chitosan that is a chitin derivative; polyamino acids such as polylysine or polyornithine; and proteins such as MBP (myelin basic protein) or histone. A method for coating the surface of a polymeric material with a composition containing a biopolymer is not particularly limited. A dip coating method is preferable.

In such a dip coating method, a base material used for the covering member is coated with the above biopolymer by being immersed in a solution containing the above biopolymer. Thereafter, as necessary, the base material is subjected to a treatment of insolubilization and neutralization of the film formed thereon, using an alkaline solution, and preferably using an aqueous sodium hydroxide solution. The base material is then washed. An example of the aforementioned for solution containing a biopolymer may be an aqueous solution obtained by dissolving a material containing a biopolymer in water. The pH of the aforementioned solution containing a biopolymer is selected, as appropriate. In order to efficiently dissolve the above biopolymer in water, it is preferably between pH 1.0 and 8.0, and more preferably between pH 2.0 and 6.5. In other words, the biopolymer is preferably in the form of an aqueous solution having a pH of 2.0 to 6.5.

The temperature applied when the base material is immersed in the aforementioned solution containing a biopolymer for coating is not particularly limited. It is preferably between 10°C and 30°C. In addition, in the above dip coating method, a base material used for the covering member is dried after coating, and also after washing. The temperature applied such drying is preferably between 40°C and 70°C, more preferably between 50°C and 60°C, and most preferably 50°C.

In the dip coating method, when chitosan is used as a biopolymer, a base material used for the covering member is immersed in a chitosan solution. The concentration of chitosan contained in the chitosan solution is between 0.01% and 1%, preferably between 0.05% and 0.8%, and most preferably 0.2%. When the aforementioned chitosan solution is an aqueous solution, such an aqueous solution is prepared by suspending chitosan in water and then adding a suitable acid such as lactic acid to the suspension, so as to dissolve the chitosan under acidic conditions. The concentration of the acid added is appropriately selected. When lactic acid is added for example, it may be added at a concentration of 0.5%. The chitosan to be used is modified with a deacetylation treatment so that the acetyl content is 40% or less, more preferably 30% or less, and most preferably 20% or less, in order to increase its solubility in an aqueous solvent. Moreover, the above base material used for the covering member is subjected to a treatment for insolubilization and neutralization of chitosan, after the treatment with a chitosan solution. The type of an alkaline solution used for the aforementioned neutralization and immobilization treatment is not particularly limited. For example, an aqueous sodium hydroxide solution is used, and the concentration of such an alkaline solution is selected, as appropriate.

The mean thickness of a coating layer comprising a composition containing a biopolymer formed by the aforementioned method can be selected, as appropriate, depending on the type of base material used, the shape thereof, the form thereof, or the usage thereof. In order to sufficiently exert the coating effects when the covering member covers a medical device and also to not lose the original properties of the base material such as flexibility, such a mean thickness is preferably between 0.002 and 1 micrometers, more preferably between 0.005 and 0.5 micrometers, and most preferably between 0.01 and 0.1 micrometers. The mean thickness of a coating layer can be calculated using the weight, that can be obtained by strict measurement, and the specific surface area of the layer coated. However, when the weight of the layer coated cannot be weighed because the layer is extremely thin, such a weight can be calculated by strictly observing the section of the coating layer by SEM (scanning electron microscope) or the like.

In another embodiment, the present invention relates to a sheet-like medical covering member produced by the method comprising: performing, as a pre-treatment, the aforementioned hydrophilic treatment on the surface of a base material used for the covering member; and then further coating the surface of the base material with the aforementioned calcium phosphate ceramics or composition comprising a biopolymer.

By the aforementioned production method, the sheet-like medical covering member of the present invention can be obtained.

In a further embodiment, the present invention relates to a highly flexible, sheet-like medical covering member for covering the surface of a medical device attached inside a living body and/or to a percutaneous site in order to prevent bacteria or outside foreign matter from invading the body, and/or in order to prevent expansion of the infection developed around the interface between the medical device and body tissues in the living body, wherein it comprises a flexible sheet-like base material which comprises a fibrous material, a porous material, or a material having a surface with many asperities, and has a thickness of 5 mm or less and a specific surface area of 100 $cm^2$/g or more, and said sheet-like base material is modified by a hydrophilic surface treatment, and/or by a coating treatment with hydroxyapatite-containing calcium phosphate ceramics or with a composition comprising a biopolymer that is positively chargeable in water.

The present invention also relates to a medical device covered by the highly flexible, sheet-like medical covering member according to the present invention.

The present invention also relates to a percutaneous driveline for an artificial heart covered by the highly flexible, sheet-like medical covering member according to the present invention.

The present invention also relates to a percutaneous catheter tube covered by the highly flexible, sheet-like medical covering member according to the present invention.

In a further embodiment, the present invention relates to a process for preparing a highly flexible, sheet-like medical covering member for covering the surface of a medical device attached inside a living body and/or to a percutaneous site comprising steps of:

- providing a flexible sheet-like base material comprising a fibrous material, a porous material, or a material having a surface with many asperities, and having a thickness of 5 mm or less and a specific surface area of 100 $cm^2$/g or more,
- subjecting said flexible sheet-like base material to a hydrophilic surface treatment, and/or to a coating treatment with hydroxyapatite-containing calcium phosphate ceramics or with a composition comprising a biopolymer that is positively chargeable in water.

A further aspect of the invention relates to use of a highly flexible, sheet-like medical covering member comprising a flexible sheet-like base material, which comprises a fibrous material, a porous material, or a material having a surface with many asperities, and has a thickness of 5 mm or less and a specific surface area of 100 $cm^2$/g or more, wherein said sheet-like base material is modified by a hydrophilic surface treatment, and/or by a coating treatment with hydroxyapatite-containing calcium phosphate ceramics or with a composition which contains a biopolymer that is positively chargeable in water, for covering the surface of a medical device attached inside a living body and/or to a percutaneous site, thereby preventing bacteria or outside foreign matter from invading the body and/or preventing the expansion of infection developed around the interface between the medical device and body tissues in the living body.

With regard to the sheet-like medical covering member of the present invention, a hydrophilic treatment, a coating treatment with calcium phosphate ceramics or a composition comprising a biopolymer, or a coating treatment with calcium phosphate ceramics or a composition comprising a biopolymer, following a hydrophilic treatment as a pretreatment, is performed on the surface of a sheet-like polymeric base material having a characteristic physical structure that has a large specific surface area as well as high flexibility, without impairing the original properties of the above material. Thus, the sheet-like medical covering member of the present invention achieves high biocompatibility, biosafety, flexibility, and superior adhesiveness to cells or other tissues. Accordingly, the surface of a medical device attached to a percutaneous site is covered with the above covering member, thereby preventing bacteria or outside foreign matter from invading the body. Further, a hydrophilic treatment on the surface of a base material generally comprises a step of chemically modifying the material surface, and thus such a hydrophilic treatment has the substantial effect of reducing the quantities of contaminants caused by microorganisms living on the surface in many cases. Accordingly, a hydrophilic treatment method applied in the present invention has an advantage for a complete sterilization step.

The present invention will be more specifically described in the following examples and comparative examples. However, the present invention is not limited by the following examples, unless the gist thereof is altered.

Example 1: Oxygen plasma treatment

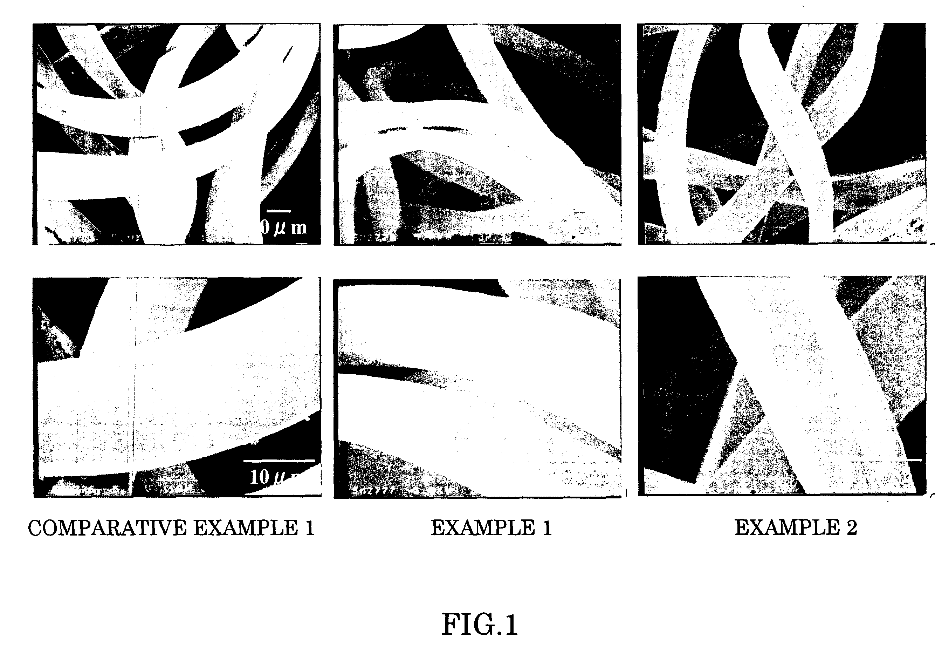

There was used a plasma treatment device (Model PD-10ND; manufactured by SAMCO, Inc., Kyoto, Tokyo) in which the ground electrode side is established at the bottom of a chamber such that the ground electrode is relatively electropositive (anodic) to the upper electrode (cathode), and such that a material to be treated disposed near the lower electrode is mainly treated with electrically neutral active species such as free radicals. A medical fabric made from polyethylene terephthalate (polyester fabric, Style 6110 DeBakey Double Velour Polyester Fabric; manufactured by C.R. BARD, Inc., AZ, USA; the specific surface area = about 2050 $cm^2$/g (calculated by the above-described formula for obtaining the specific surface area, based on the assumption that the geometrical configuration of the fibrous material is cylindrical, using its typical diameter measured by SEM image of 15 micrometers and its density of 1.3 g/cm$^3$: SSA = 4/(Dxp) = 4/(0.0015×1.3)= 2051 cm$^2$/g)) was cut into a suitable size, and it was used as a base material (the material to be treated). The medical fabric was spread on the lower ground electrode (anode), and a plasma treatment was carried out at a distance between electrodes of 55 mm, under an oxygen gas current comprising a gas flow of 150 ml/min., and under a partial pressure of oxygen of 66.5 Pa. The temperature of the base was set at 30°C, and the electric discharge output was set at 150 W. The plasma treatment was performed for 5 minutes on each of the front and back sides of the polyester fabric. By the above treatment, the sheet-like medical covering member of the present invention was produced. Using photographs obtained from an SEM (ERA-4000; manufactured by ELIONIX Inc., Tokyo, Japan), the surface of the produced sheet-like medical covering member was observed. As a result, deterioration of the surface by such a surface treatment was not observed (FIG. 1).

Example 2: Alkali reduction treatment

A medical fabric made from polyethylene terephthalate (polyester fabric, Style 6110 DeBakey Double Velour Polyester Fabric; manufactured by C.R. BARD, Inc., AZ, USA; the specific surface area = about 2050 cm$^2$/g (calculated as described in Example 1)) was cut into a suitable size, and it was then dried at 50°C for 3 hours. Thereafter, the dry weight of the above polyester fabric was measured. An aqueous NaOH solution was prepared in a plastic container, and it was then left at rest in a thermostatic bath that was adjusted to be between 85°C and 90°C. When the temperature of the solution in the container reached 80±5°C, the above polyester fabric was immersed in the above solution. After a certain reaction time had passed, the above polyester fabric was washed with distilled water 3 or 4 times, and it was then immersed in 1.0 N HCl for 30 minutes. The concentration of NaOH used in the reaction was 1.0 N, and the reaction time was 30 minutes. The polyester fabric was further washed with distilled water 3 or 4 times. Thereafter, it was confirmed using a pH test paper that the washing solution became neutral. Thereafter, the polyester fabric was dried at 50°C overnight, and the dry weight thereof was then measured. The reduction rate was then measured. As a result, the reduction rate was found to be approximately 2%. By the above treatment, the sheet-like medical covering member of the present invention was produced. The surface of the produced sheet-like medical covering member was observed using an SEM (ERA-4000; manufactured by ELIONIX Inc., Tokyo, Japan). As a result, deterioration of the surface by such a surface treatment was not observed (FIG. 1).

Example 3: Hydroxyapatite coating

A medical fabric made from polyethylene terephthalate (polyester fabric, Style 6110 DeBakey Double Velour Polyester Fabric; manufactured by C.R. BARD, Inc., AZ, USA; the specific surface area = about 2050 cm$^2$/g (calculated as described in Example 1)) was cut into a suitable size, and it was then placed on a suitable grid without being overlapped. Another grid was then placed thereon, so that the above polyester fabric was sandwiched between the two grids. The following 4 types of solutions (A) to (D) were prepared:

(A) 3.329 g of CaCl$_2$ (Wako Pure Chemical Industries, Ltd., Osaka, Japan) was dissolved in 150 ml of solution containing 50 mmol/l Tris-HCl (manufactured by ROCKLAND Immunochemicals, Inc., PA, USA) in a 500-ml beaker, so as to prepare a solution containing 200 mmol/l CaCl$_2$;
(B) 200 to 300 ml of distilled water was poured into a 500-ml beaker (for washing CaCl$_2$);
(C) 2.555 g of Na$_2$HPO$_4$ (Wako Pure Chemical Industries, Ltd., Osaka, Japan) was dissolved in 150 ml of distilled water in a 500-ml beaker, so as to prepare a solution containing 120 mmol/l Na$_2$HPO$_4$; and
(D) 200 to 300 ml of distilled water was poured into a 500-ml beaker (for washing Na$_2$HPO$_4$).

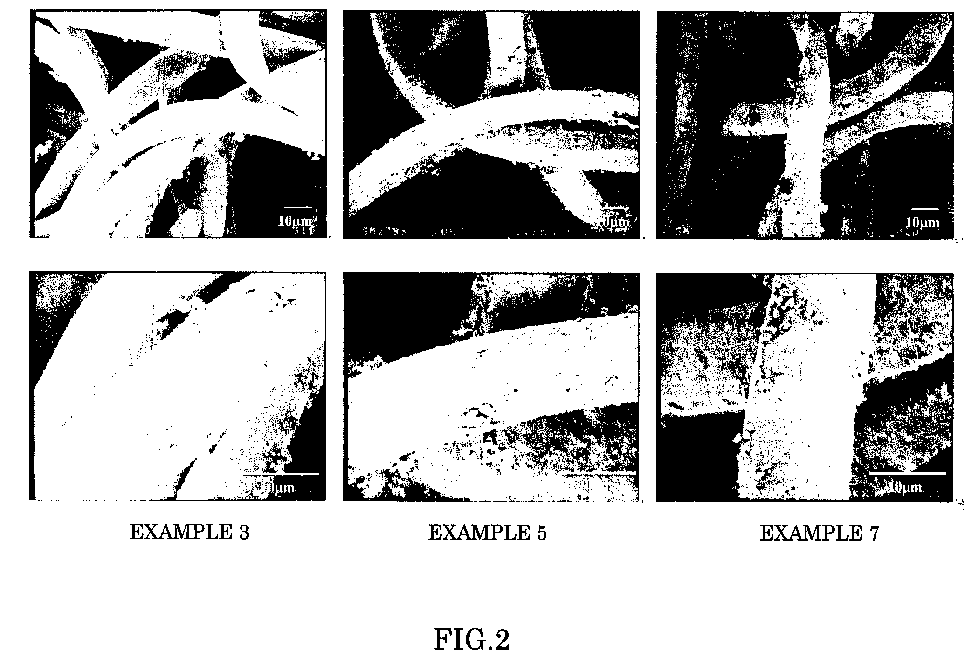

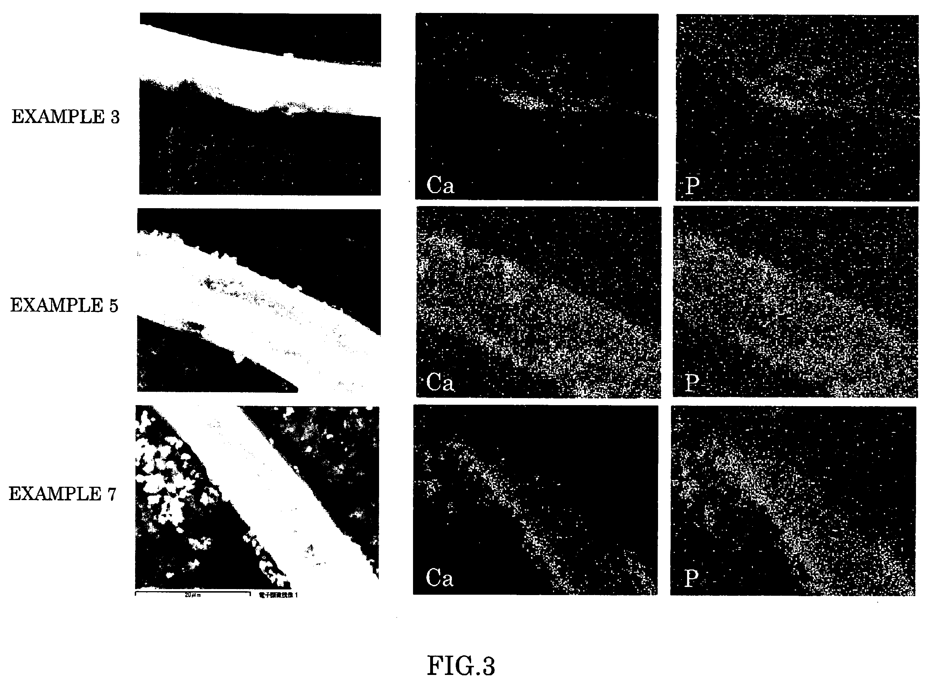

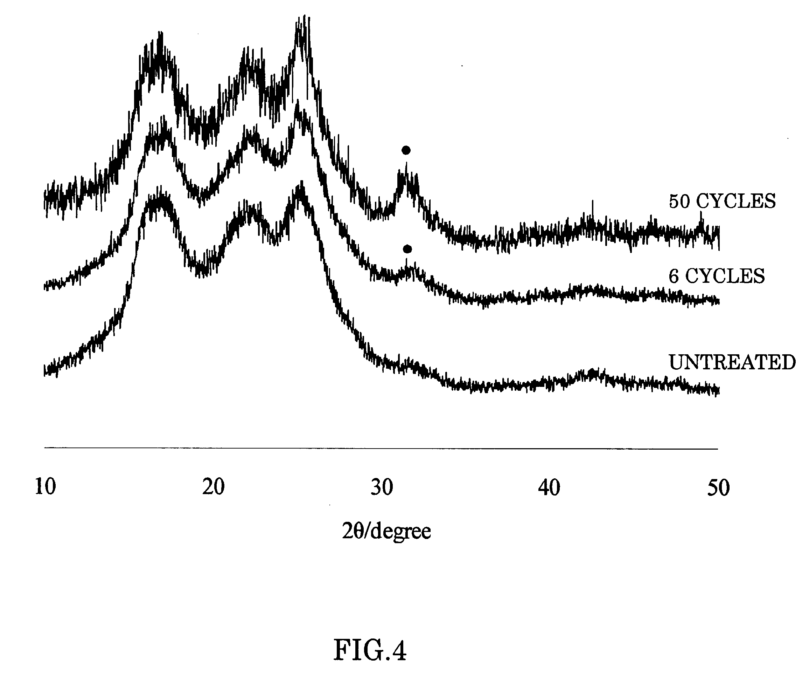

Thereafter, these beakers were gently placed in a thermostatic bath (THERMAL RABO TR-2A; manufactured by AS ONE Corp., Osaka, Japan) filled with water, the temperature of which was adjusted to between 37°C and 38°C. The temperature of the solution in each beaker was measured with a thermometer, and when the temperature reached 37°C, a coating treatment was initiated. The coating treatment was carried out by immersing the polyester fabric sandwiched between the two grids in the aforementioned solutions (A), (B), (C), and (D), in this order, for 60 seconds each. When the polyester fabric was immersed in each solution, the previous solution attached on the polyester fabric was sufficiently removed, and the fabric was then immersed in the next solution. Immersion of the polyester fabric in the solutions (A), (B), (C), and (D), in this order, is defined as 1 cycle, and 4 cycles were performed as a coating treatment. After completion of the coating treatment, the above polyester fabric was placed in a dryer (Hot Air Sterilizer HE-102; manufactured by Sakura Seiki Corp., Tokyo, Japan), and it was dried at 50°C to 60°C. Thereafter, the above polyester fabric was immersed in distilled water, followed by ultrasonic washing (approximately 1 minute × 3 times). The polyester fabric was placed in a dryer again, and it was then dried at 50°C to 60°C. By the above treatment, the sheet-like medical covering member of the present invention was produced. The surface of the produced sheet-like medical covering member was observed using an SEM (ERA-4000; manufactured by ELIONIX Inc., Tokyo, Japan) and an SEM combined with the energy dispersive X-ray microanalysis (SEM-EDX) (Type N; manufactured by Hitachi Science Systems, Ltd., Ibaragi, Japan). As a result, a thin uniform coating layer comprising many fine hydroxyapatite particles, and calcium and phosphorus that were uniformly distributed, were observed (FIGS. 2 and 3). In addition, as a result of measurement using a wavelength dispersive X-ray fluorescent spectrometer (PW2400; manufactured by Philips, Netherland), the content of calcium was found to be approximately 0.99% by weight, and the content of phosphorus was found to be approximately 0.50% by weight. The thickness of a hydroxyapatite coating layer was calculated based on these results using each atomic weight ratio. The thickness was found to be approximately 0.006 micrometers. Moreover, measurement was further carried out using a X-ray diffractometer (2035; manufactured by Rigaku Corp., Tokyo, Japan). (In order to increase detection sensitivity, a covering member, on which 6 or 50 immersion cycles had been carried out, was used for the X-ray diffraction measurement). As a result, it was confirmed that the coating layer on the surface of the sheet-like medical covering member was a hydroxyapatite layer having a characteristic peak at 32 degrees of the $2\theta$ value (FIG. 4).

Example 4: Chitosan coating

A medical fabric made from polyethylene terephthalate (polyester fabric, Style 6110 DeBakey Double Velour Polyester Fabric; manufactured by C.R. BARD, Inc., AZ, USA; the specific surface area = about 2050 $cm^2/g$ (calculated as described in Example 1)) was cut into a suitable size, and it was then dried at 50°C for 3 hours. Thereafter, the dry weight of the above polyester fabric was measured. The above polyester fabric was placed in a suitable container. The following solutions (A) and (B) were prepared:

(A) An aqueous chitosan solution: Chitosan (UltrasanCH01; manufactured by Biosyntech, Inc., Canada) having a deacetylation degree of 94.6% was suspended in distilled water, and lactic acid (the concentration of acid in the solution: 0.5%) was then added thereto. Thereafter, the mixture was fully stirred, until chitosan was completely dissolved therein.
(B) An aqueous 0.1 N NaOH solution: 0.2 g of NaOH was dissolved in 50 ml of distilled water, resulting in a concentration of 0.1 N.

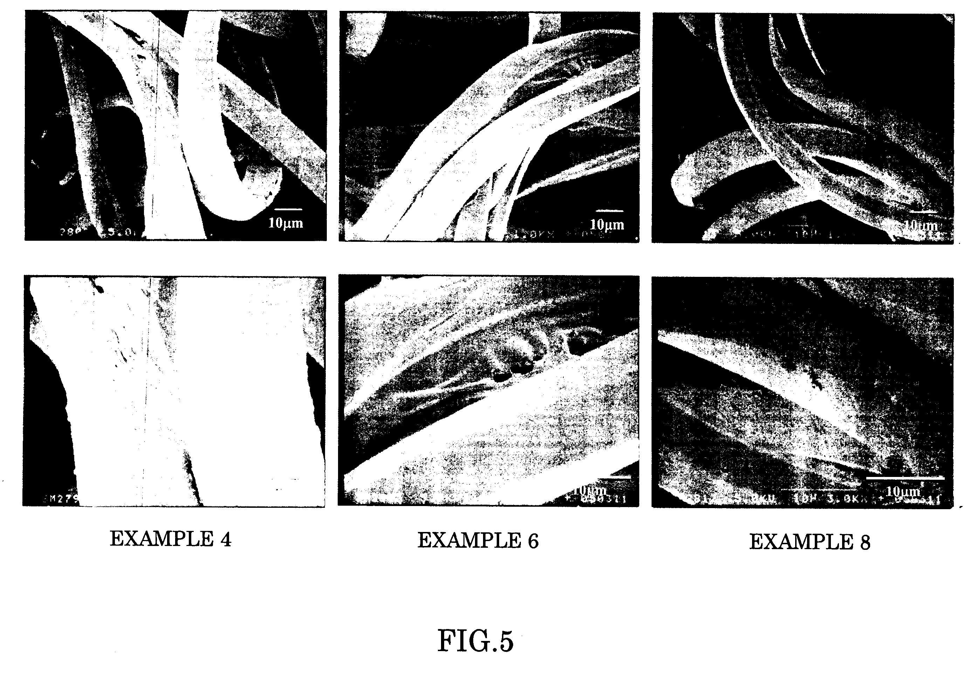

The concentration of the aqueous chitosan solution was adjusted so as to prepare an aqueous 0.2% chitosan solution. A coating treatment was carried out by impregnating the polyester fabric with the aqueous chitosan solution (to such an extent that the polyester fabric was sufficiently soaked in the solution). The above polyester fabric was then dried in a dryer (Hot Air Sterilizer HE-102; manufactured by Sakura Seiki Corp., Tokyo, Japan). Thereafter, the polyester fabric was immersed in an aqueous 0.1 N NaOH solution for the purpose of insolubilization and neutralization of chitosan. After NaOH had been removed, the above polyester fabric was repeatedly washed with distilled water. Thereafter, it was confirmed using a pH test paper that the washing solution became neutral. Thereafter, the polyester fabric was dried at 50°C overnight, and the dry weight thereof was then measured. By the above treatment, the sheet-like medical covering member of the present invention was produced. The amount of chitosan coated was calculated based on the dry weights of the polyester fabric changed before and after the aforementioned treatment. As a result, the coating amount was found to be 1.24%. This amount was converted into the thickness of chitosan. As a result, the thickness was found to be approximately 0.04 micrometers. The surface of the produced sheet-like medical covering member was observed using an SEM (ERA-4000; manufactured by ELIONIX Inc., Tokyo, Japan). As a result, a thin uniform coating layer was confirmed (FIG. 5).

Example 5: Hydroxyapatite coating after oxygen plasma treatment

A medical fabric made from polyethylene terephthalate (polyester fabric, Style 6110 DeBakey Double Velour Polyester Fabric; manufactured by C.R. BARD, Inc., AZ, USA; the specific surface area = about 2050 $cm^2/g$ (calculated as described in Example 1)) was cut into a suitable size, and it was then subjected to the oxygen plasma treatment described in Example 1. Thereafter, the polyester fabric was subjected to the hydroxyapatite coating described in Example 3. By the above treatments, the sheet-like medical covering member of the present invention was produced. The surface of the produced sheet-like medical covering member was observed in the same manner as in Example 3. As a result, a thin uniform coating layer comprising many fine hydroxyapatite particles, and calcium and phosphorus that were uniformly distributed, were observed (FIGS. 2 and 3). Thereafter, the sheet-like covering member was subjected to the same quantitative analysis for calcium and phosphorus as in Example 3. As a result, the content of calcium was found to be approximately 1.93% by weight, and the content of phosphorus was found to be approximately 1.09% by weight. The thickness of a hydroxyapatite coating layer was calculated based on these results using each atomic weight ratio.

The thickness was found to be approximately 0.011 micrometers.

Example 6: Chitosan coating after oxygen plasma treatment

A medical fabric made from polyethylene terephthalate (polyester fabric, Style 6110 DeBakey Double Velour Polyester Fabric; manufactured by C.R. BARD, Inc., AZ, USA; the specific surface area = about 2050 $cm^2$/g (calculated as described in Example 1)) was cut into a suitable size, and it was then subjected to the oxygen plasma treatment described in Example 1. Thereafter, the polyester fabric was subjected to the chitosan coating described in Example 4. By the above treatments, the sheet-like medical covering member of the present invention was produced. The increased amount of chitosan coated was calculated based on the dry weights of the polyester fabric changed before and after the aforementioned treatment. As a result, the increased coating amount was found to be 1.33%. This amount was converted into the thickness of chitosan. As a result, the thickness was found to be approximately 0.05 micrometers. The surface of the produced sheet-like medical covering member was observed using an SEM (ERA-4000; manufactured by ELIONIX Inc., Tokyo, Japan). As a result, a thin uniform coating layer was observed (FIG. 5).

Example 7: Hydroxyapatite coating after alkali reduction treatment

A medical fabric made from polyethylene terephthalate (polyester fabric, Style 6110 DeBakey Double Velour Polyester Fabric; manufactured by C.R. BARD, Inc., AZ, USA; the specific surface area = about 2050 $cm^2$/g (calculated as described in Example 1)) was cut into a suitable size, and it was then subjected to the alkali reduction treatment described in Example 2. Thereafter, the polyester fabric was subjected to the hydroxyapatite coating described in Example 3. By the above treatments, the sheet-like medical covering member of the present invention was produced. The surface of the produced sheet-like medical covering member was observed in the same manner as in Example 3. As a result, a thin uniform coating layer comprising many fine hydroxyapatite particles, and calcium and phosphorus that were uniformly distributed, were observed (FIGS. 2 and 3). Thereafter, the sheet-like covering member was subjected to the same quantitative analysis for calcium and phosphorus as in Example 3. As a result, the content of calcium was found to be approximately 1.82% by weight, and the content of phosphorus was found to be approximately 1.13% by weight. The thickness of a hydroxyapatite coating layer was calculated based on these results using each atomic weight ratio. The thickness was found to be approximately 0.011 micrometers.

Example 8: Chitosan coating after alkali reduction treatment

A medical fabric made from polyethylene terephthalate (polyester fabric, Style 6110 DeBakey Double Velour Polyester Fabric; manufactured by C.R. BARD, Inc., AZ, USA; the specific surface area = about 2050 $cm^2$/g (calculated as described in Example 1)) was cut into a suitable size, and it was then subjected to the alkali reduction treatment described in Example 2. Thereafter, the polyester fabric was subjected to the chitosan coating described in Example 4. The amount of chitosan coating was calculated based on the dry weights of the polyester fabric changed before and after the aforementioned treatment. As a result, the coating amount was found to be 1.29%. This amount was converted into the thickness of chitosan. As a result, the thickness was found to be approximately 0.04 micrometers. By the above treatments, the sheet-like medical covering member of the present invention was produced. The surface of the produced sheet-like medical covering member was observed using an SEM (ERA-4000; manufactured by ELIONIX Inc., Tokyo, Japan). As a result, a thin uniform coating layer was observed (FIG. 5).

Comparative example 1

The medical fabric made from polyethylene terephthalate (polyester fabric, Style 6110 DeBakey Double Velour Polyester Fabric; manufactured by C.R. BARD, Inc., AZ, USA; the specific surface area = about 2050 $cm^2$/g (calculated as described in Example 1)), which was cut into a suitable size and was used in Examples 1 to 8, was not treated. Thus, the untreated medical fabric was evaluated in the same manner as in the above examples.

Test example 1 (cell adhesion and cell growth test)

The sheet-like medical covering member obtained in each of Examples 1 to 8 and Comparative example 1 was subjected to an *in vitro* cell adhesion and cell growth test. The cell adhesion and cell growth test was performed using TetraColor ONE (800560; Cell Proliferation Assay System; manufactured by SEIKAGAKU Corp., Tokyo, Japan) according to the manufacture's protocol. As cells, human embryo fibroblasts (HE-49) were used. As a medium, a medium produced by adding 10% fetal bovine serum (FBS) to a DMEM medium [Dulbecco's Modified Eagle's Medium (manufacture by Invitrogen Corp., Tokyo, Japan), 100 mg/ml streptomycin (for biochemical use; manufactured by Wako Pure Chemical Industries, Ltd., Osaka, Japan), 100 units/ml penicillin (for biochemical use; manufactured by Wako Pure Chemical Industries, Ltd., Osaka, Japan), and 1.4 g/l $NaHCO_3$ (special grade; manufactured by Wako Pure Chemical Industries, Ltd., Osaka, Japan); pH 7.2] was used. The cells were cultured at 37°C in the presence of 5% $CO_2$ ($CO_2$ INCUBATOR IT-63; manufactured by YAMATO SCIENTIFIC, Tokyo, Japan). The above sheet-like medical covering member was placed on the bottom of a 96-well microplate (manufactured by Nalge Nunc International, NY, USA), and it was impregnated with approximately 0.1 ml of the DMEM medium. Thereafter, using the DMEM medium, the above covering member was inoculated with 0.1 ml each of an HE-49 suspension that had been adjusted to be a concentration of approximately 1.0 to 1.5 $\times$ $10^4$ cells/0.1 ml. One day after the culture, each of the sheet-like covering member was gently removed, and it was then transferred to a 24-well microplate (manufactured by Nalge Nunc International, NY, USA) filled with the medium. Three days after the culture, the medium was exchanged with a fresh medium.

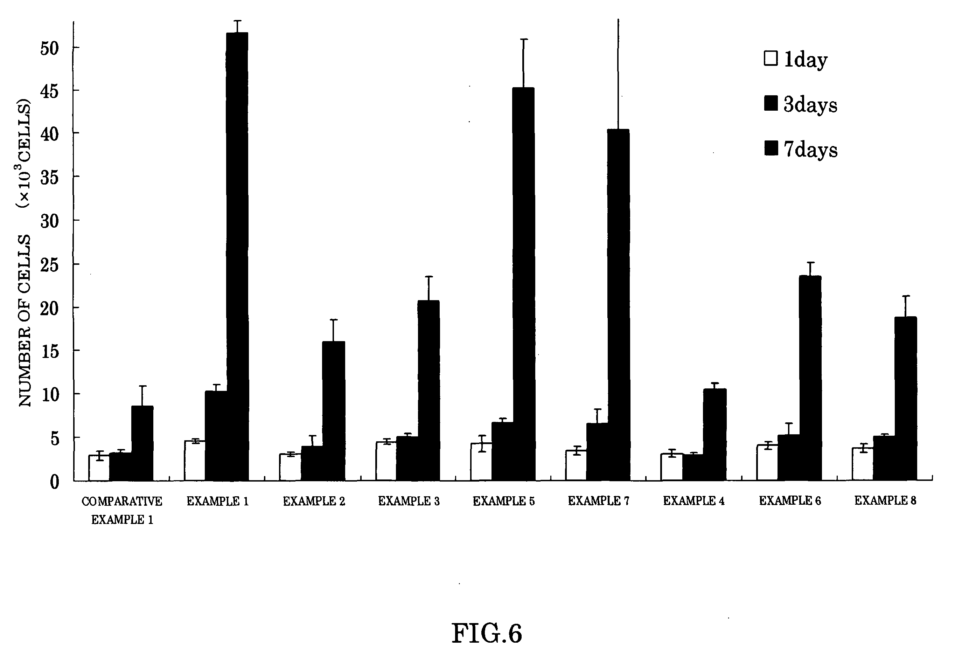

1, 3, and 7 days after the culture, each of the sheet-like covering members were gently removed, and they were then washed by shaking in a serum free medium, so as to eliminate unattached cells. Thereafter, the sheet-like covering member was transferred to a new 96-well microplate. To the above sheet-like covering member, 0.1 ml of a reaction solution obtained by mixing a serum free DMEM medium and TetraColor ONE at a mixing ratio of 9 : 1 was added. The obtained mixture was incubated for 2 hours under conditions consisting of 37°C and 5% $CO_2$. Subsequently, 0.1 ml of the resultant solution was transferred to a 96-well Immuno Microplate (manufactured by Nalge Nunc International, NY, USA), and the absorbance at 450 nm was then measured (target wavelength: 630 nm), using a microplate reader (Model 550; manufactured by Bio-Rad Laboratories, Inc.,CA, USA). The number of cells on each sheet-like covering member was calculated using the obtained absorbance. The measurement results are shown in FIG. 6.

Test example 2 (Evaluation of adhesive properties in animal experiment)

The sheet-like medical covering members of the present invention obtained in Examples 1 and 6 were used. Also, the sheet-like medical covering member obtained in Comparative Example 1 was used to compare treated base materials with an untreated base material. These sheet-like covering members were cut into the form of straps with a size of approximately 1 cm $\times$ 5 cm. Thereafter, the obtained straps were subcutaneously embedded in the back of two calves (Jersey; male; body weight: 67.0 kg; supplier: Fischer). 1, 2, and 4 weeks later, each strap was removed together with surrounding tissues, and they were then fixed with 10% formalin. Thereafter, it was embedded in paraffin, and sections were produced with a microtome. The section was stained with hematoxylin and eosin (HE), and histological observation was carried out under a microscope. Implantation of the strap into the animal and histological observation were carried out in cooperation with an American Institution [McGowan Institute For Regenerative Medicine, University of Pittsburgh, NAMSA (North American Science Associates, Inc.)]. The adhesive state of cells on each sheet-like covering member was evaluated in terms of regeneration of surrounding tissues (entering of tissues or cells into the fabric and the density thereof), migration of inflammatory cells (entering of inflammatory cells such as macrophages or lymphocytes into the fabric), and the adhesiveness of the fabric to tissues (adhesion at the interface between the fabric and tissues) (Table 1). As a result of the comprehensive evaluation of these factors, it was found that the sheet-like medical covering members of the present invention obtained in Examples 1 and 6 were superior to the untreated base material in terms of the adhesiveness to tissues.

Table 1 - Results of evaluation on the adhesiveness of the sheet-like medical covering members obtained in Examples 1 and 6 to tissues in animal experiment:

| sheet-like medical covering member | Implantation period | Regeneration of surrounding tissues | Migration of inflammatory cells | Adhesiveness of fabric to tissues |
|---|---|---|---|---|
| Comparative example 1 [Untreated] | 1 week | M | M | P |
| | 2 weeks | P | P | P |
| | 4 weeks | P | M | P |
| Example 1 | 1 week | G | - | G |
| | 2 weeks | E | - | G |
| | 4 weeks | E | - | G |

(continued)

| sheet-like medical covering member | Implantation period | Regeneration of surrounding tissues | Migration of inflammatory cells | Adhesiveness of fabric to tissues |
|---|---|---|---|---|
| Example 6 | 1 week | E | E | E |
| | 2 weeks | E | M | E |
| | 4 weeks | E | M | M |
| E: High, G: Moderately high, M: Moderate, P: Slightly moderate or low, -; No data | | | | |

Test example 3 (Hydrophilic test)

The sheet-like medical covering members obtained in Examples 1 to 8 and Comparative Example 1 were cut into sections with a size of approximately 1 cm × 2 cm. The obtained sections were used as samples. The time required for water to permeate from the bottom to the top of such a sample was measured. As a result, it was found that no water permeated into the covering member of Comparative Example 1 and thus the above covering member exhibited water repellency, whereas water permeated into the covering members of Examples 1 to 8 and thus these covering members exhibited hydrophilicity (Table 2).

Table 2 - Results of hydrophilic test on the sheet-like medical covering members obtained in Examples 1 to 8 and Comparative Example 1:

| sheet-like medical covering member | Permeation time (sec.) | | | Average (sec.) | Evaluation |
|---|---|---|---|---|---|
| | Measurement 1 | Measurement 2 | Measurement 3 | | |
| Comparative Example 1 [Untreated] | >100 | >100 | >100 | >100 | Zero permeation (water repellency) |
| Example 1 [Oxygen plasma treatment] | 3 | 3 | 3 | 3 | Immediate permeation |
| Example 2 [Alkali reduction treatment] | 52 | 34 | 46 | 44 | Permeation |
| Example 3 [Hydroxyapatite coating] | 77 | 67 | 46 | 44 | Permeation |
| Example 5 [Hydroxyapatite coating after oxygen plasma treatment] | 1 | 1 | 2 | 1 | Immediate permeation |
| Example 7 [Hydroxyapatite coating after alkali reduction treatment] | 2 | 2 | 2 | 2 | Immediate permeation |
| Example 4 [Chitosan coating] | 4 | 4 | 3 | 4 | Quick permeation |

(continued)

| sheet-like medical covering member | Permeation time (sec.) | | | Average (sec.) | Evaluation |
|---|---|---|---|---|---|
| | Measurement 1 | Measurement 2 | Measurement 3 | | |
| Example 6 [Chitosan coating after oxygen plasma treatment] | 2 | 1 | 2 | 2 | Immediate permeation |
| Example 8 [Chitosan coating after alkali reduction treatment] | 2 | 3 | 2 | 2 | Immediate permeation |
| Numbers less than one second have been rounded off. | | | | | |

Test Example 4 (Antibacterial test)

The sheet-like medical covering members obtained in Examples 1, 5, and 6, and Comparative Example 1 were subjected to an antibacterial test. The test was carried out in accordance with JIS L1902 "Quantitative antibacterial test method for fiber products". First, the sheet-like medical covering members obtained in Examples 1, 5, and 6, and Comparative Example 1 were inoculated with test bacteria (*Staphylococcus aureus,* manufactured by MicroBioLogics, MN, USA). 18 hours later, the growth value (F), the bacteriostatic value (S), and the bactericidal value (L) were obtained. Thereafter, antibacterial properties were evaluated.

Growth value (F) = Log II - Log I
Bacteriostatic value (S) = Log II - Log III to V
Bactericidal value (L) = Log I - Log III to V
(I - The number of surviving cells immediately after inoculation in Comparative Example 1
II - The number of surviving cells 18 hours after culture in Comparative Example 1
III to V - The number of surviving cells 18 hours after culture in Examples 1, 5, and 6)

The test holds when F > 1.5 with regard to the growth value (F). The bacteriostatic value (S) is a value indicating the effect of inhibiting the growth of bacteria. When S > 2.2, it indicates the presence of bacteriostatic activity. The bactericidal value (L) is a value indicating the effect of killing bacteria. When L > 0, there is a presence of bactericidal activity. Covering members having both bacteriostatic and bactericidal activities were evaluated to have antibacterial properties. As a result of the evaluation, it was found that the sheet-like medical covering member obtained in Example 1 has no antibacterial properties, whereas the sheet-like medical covering members obtained in Examples 5 and 6 have antibacterial properties (Table 3)

Table 3 - Results of antibacterial test on the sheet-like medical covering members obtained in Examples 1, 5, and 6, and Comparative Example 1

| | sheet-like medical covering member (culture time) | Number of surviving cells (mean value) (cells/ml) | Growth value (F) | Bacteriostatic value (S) | Bactericidal value (L) |
|---|---|---|---|---|---|
| I | Comparative Example 1 [untreated (0 hour)] | $7.7 \times 10^3$ | | | |

(continued)

| | sheet-like medical covering member (culture time) | Number of surviving cells (mean value) (cells/ml) | Growth value (F) | Bacteriostatic value (S) | Bactericidal value (L) |
|---|---|---|---|---|---|
| II | Comparative example 1 [untreated (18 hours)] | $5.5 \times 10^5$ | 1.9 | | |
| III | Example 1 (18 hours) | $9.6 \times 10^5$ | | -0.2 | -2.1 |
| IV | Example 5 (18 hours) | $>2.0 \times 10^2$ | | **3.4** | **1.6** |
| V | Example 6 (18 hours) | $>2.0 \times 10^2$ | | **3.4** | **1.6** |
| $> 2.0 \times 10^2$; not detected | | | | | |

The sheet-like medical covering member of the present invention is uniformly coated with hydroxyapatite comprising many fine particles as is clear from the results shown in FIGS. 2 and 3, or it is thinly and uniformly coated with chitosan as is clear from the results shown in FIG. 5. Thus, the sheet-like medical covering member of the present invention is modified by a surface treatment and/or a coating treatment, which do not impair the original properties of the base material. Hence, the sheet-like medical covering member of the present invention can adopt a flexible structure depending on the configuration of a medical device and can maintain stable and strong coating in a living body. Accordingly, the sheet-like medical covering member of the present invention can sufficiently exert its coating effect. As is apparent from the results of Test examples 1 to 3, the sheet-like medical covering member of the present invention is extremely excellent in terms of hydrophilicity, and adhesiveness to cells or other tissues, and cell growth and adhesive properties. In addition, as is clear from the results of Test example 4, the sheet-like medical covering member of the present invention also has excellent antibacterial properties. Accordingly, the sheet-like medical covering member of the present invention achieves high biocompatibility, bio-safety, flexibility, excellent adhesiveness to cells or other tissues, and antibacterial properties, by the combined use of a sheet-like polymeric base material having a characteristic physical structure that has a large specific surface area as well as high flexibility, with a hydrophilic treatment and/or a coating treatment with hydroxyapatite or chitosan performed on the surface of the material. Thus, the surface of a medical device attached inside a living body and/or to a percutaneous site is covered with the aforementioned covering member, so as to prevent bacteria or foreign matter from the outside invading the body, and/or so as to prevent expansion of the infection developed around the interface between the medical device and body tissues in a living body. Further, a hydrophilic treatment on the surface of a base material generally comprises a step of chemically modifying the material surface, and thus such a hydrophilic treatment has the substantial effect of reducing the quantities of contaminants caused by microorganisms living on the surface in many cases. Accordingly, a hydrophilic treatment method applied in the present invention has an advantage for a complete sterilization step.

The covering member of the present invention has high biocompatibility, bio-safety, adhesiveness to cells or other tissues, antibacterial properties, and flexibility. Accordingly, the covering member is useful as a covering member for various medical devices or medical materials attached inside a living body or to a percutaneous site, and particularly for a medical device or medical material that is fixed and stays inside a living body or fixed to a percutaneous site over a long period of time.

**Claims**

1. A highly flexible, sheet-like medical covering member for covering the surface of a medical device attached inside a living body and/or to a percutaneous site in order to prevent bacteria or outside foreign matter from invading the body, and/or in order to prevent expansion of the infection developed around the interface between the medical device and body tissues in the living body, wherein it comprises a flexible sheet-like base material which comprises a fibrous material, a porous material, or a material having a surface with many asperities, and has a thickness of 5 mm or less and a specific surface area of 100 cm$^2$/g or more, and said sheet-like base material is modified by a hydrophilic surface treatment, and/or by a coating treatment with hydroxyapatite-containing calcium phosphate ceramics or with a composition comprising a biopolymer that is positively chargeable in water.

2.  A highly flexible, sheet-like medical covering member according to claim 1, wherein the sheet-like base material comprises a woven fabric, a nonwoven fabric, or a knitted fabric, each made of synthetic fibers.

3.  A highly flexible, sheet-like medical covering member according to claim 2, wherein the woven fabric, the nonwoven fabric, and the knitted fabric are made of polyester fibers, polyolefin fibers, or fluorocarbon resin fibers.

4.  A highly flexible, sheet-like medical covering member according to claim 1, wherein the sheet-like base material is a porous sheet-like material made of a polyolefin or a fluorocarbon resin.

5.  A highly flexible, sheet-like medical covering member according to any one of claims 1 to 4, wherein the sheet-like base material is subjected to the hydrophilic surface treatment before the coating treatment.

6.  A highly flexible, sheet-like medical covering member according to any one of claims 1 to 5, wherein the hydrophilic surface treatment and/or the coating treatment also provides antibacterial properties on the surface of said covering member.

7.  A highly flexible, sheet-like medical covering member according to any one of claims 1 to 6, wherein the surface treatment is a plasma treatment which essentially comprises being carried out in an oxygen, argon or ammonia-containing atmosphere and using a plasma treatment device configured such that the main portion of active plasma species substantially having no electric charge plays a dominant role in a denaturing process mechanism for treating the material.

8.  A highly flexible, sheet-like medical covering member according to claim 7, wherein the plasma treatment is carried out for 2 to 20 minutes in an oxygen-containing atmosphere having a partial pressure of 5 to 500 Pa.

9.  A highly flexible, sheet-like medical covering member according to any one of claims 1 to 6, wherein the surface treatment is an alkali reduction treatment.

10. A highly flexible, sheet-like medical covering member according to any one of claims 1 to 6, wherein the surface treatment is an ultraviolet ray irradiation treatment in the presence of ozone, or is an corona discharge treatment.

11. A highly flexible, sheet-like medical covering member according to any one of claims 1 to 10, wherein the mean thickness of the coating layer formed by the coating treatment using hydroxyapatite-containing calcium phosphate ceramics is between 0.001 and 0.5 micrometers.

12. A highly flexible, sheet-like medical covering member according to any one of claims 1 to 11, wherein the coating treatment using hydroxyapatite-containing calcium phosphate ceramics is carried out by a solution immersion method, a suspension immersion method, or a solution alternate immersion method.

13. A highly flexible, sheet-like medical covering member according to claim 12, wherein, in the solution alternate immersion method, a calcium solution containing 100 to 300 mmol/l of calcium ions and a phosphoric acid solution containing 100 to 300 mmol/l of phosphate ions are used to form said hydroxyapatite-containing calcium phosphate ceramics.

14. A highly flexible, sheet-like medical covering member according to claim 12, wherein, in the suspension immersion method, a suspension comprising calcined hydroxyapatite having a mean particle size of 1 micrometer or less is used.

15. A highly flexible, sheet-like medical covering member according to any one of claims 1 to 10, wherein the mean thickness of the coating layer formed by the coating treatment using a composition comprising a biopolymer that is positively chargeable in water is between 0.002 and 1.0 micrometers.

16. A highly flexible, sheet-like medical covering member according to any one of claims 1 to 15, wherein the coating treatment using a composition comprising a biopolymer that is positively chargeable in water is carried out by a dip coating method.

**17.** A highly flexible, sheet-like medical covering member according to any one of claims 1 to 6, 15, and 16, wherein the biopolymer is chitosan which is modified with a deacetylation treatment so that the acetyl content is 20% or less.

**18.** A highly flexible, sheet-like medical covering member according to claim 17, wherein the modified chitosan is in the form of an aqueous solution having a pH of 2.0 to 6.5.

**19.** A medical device covered by a highly flexible, sheet-like medical covering member according to any one of claims 1 to 18.

**20.** A medical device according to claim 19, wherein the device is a percutaneous driveline for an artificial heart.

**21.** A medical device according to claim 19, wherein the device is a percutaneous catheter tube.

**22.** A process for preparing a highly flexible, sheet-like medical covering member for covering the surface of a medical device attached inside a living body and/or to a percutaneous site comprising steps of:

- providing a flexible sheet-like base material comprising a fibrous material, a porous material, or a material having a surface with many asperities, and having a thickness of 5 mm or less and a specific surface area of 100 cm$^2$/g or more,
- subjecting said flexible sheet-like base material to a hydrophilic surface treatment, and/or to a coating treatment with hydroxyapatite-containing calcium phosphate ceramics or with a composition comprising a biopolymer that is positively chargeable in water.

**23.** Use of a highly flexible, sheet-like medical covering member comprising a flexible sheet-like base material, which comprises a fibrous material, a porous material, or a material having a surface with many asperities, and has a thickness of 5 mm or less and a specific surface area of 100 cm$^2$/g or more, wherein said sheet-like base material is modified by a hydrophilic surface treatment, and/or by a coating treatment with hydroxyapatite-containing calcium phosphate ceramics or with a composition which contains a biopolymer that is positively chargeable in water, for covering the surface of a medical device attached inside a living body and/or to a percutaneous site, thereby preventing bacteria or outside foreign matter from invading the body and/or preventing the expansion of infection developed around the interface between the medical device and body tissues in the living body.

COMPARATIVE EXAMPLE 1          EXAMPLE 1          EXAMPLE 2

FIG.1

EXAMPLE 3          EXAMPLE 5          EXAMPLE 7

FIG.2

EXAMPLE 3

EXAMPLE 5

EXAMPLE 7

Ca

Ca

Ca

P

P

P

FIG.3

FIG.4

EP 1 754 495 A2

EXAMPLE 4                 EXAMPLE 6                 EXAMPLE 8

## FIG.5

FIG.6

EP 1 754 495 A2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004097267 A
- JP 6293504 A
- JP 2004283324 A
- JP 6293505 A
- JP 6293507 A
- JP 2000327314 A

- JP 2579610 B
- JP 7258972 A
- WO 9511666 A
- JP 2004131600 A
- JP 100 A

**Non-patent literature cited in the description**

- **MCGOWAN.** Institute For Regenerative Medicine. North American Science Associates, Inc,